# EUROPEAN PATENT SPECIFICATION

(11) **EP 3 248 015 B1**
(45) Date of publication and mention of the grant of the patent: **14.05.2025**
(21) Application number: 16702206.0
(22) Date of filing: 21.01.2016
(51) Int. Cl.: G01N 33/68, G01N 33/96

(54) **COMPOUNDS AND METHODS FOR THE DETECTION OF CALPROTECTIN**
VERBINDUNGEN UND VERFAHREN FÜR DEN NACHWEIS VON CALPROTECTIN
COMPOSÉS ET PROCÉDÉS PERMETTANT LA DÉTECTION DE LA CALPROTECTINE

(30) Priority: 23.01.2015 EP 15000207
(43) Date of publication of application: 29.11.2017
(73) Proprietor: Westfälische Wilhelms-Universität Münster, 48149 Münster (DE)
(72) Inventor: ROTH, Johannes, 48153 Münster (DE); VOGL, Thomas, 48161 Münster (DE)
(74) Representative: Schiweck Weinzierl Koch Patentanwälte Partnerschaft mbB
(86) International application number: PCT/IB2016/050288
(87) International publication number: WO 2016/116881

(56) References cited:
- WO-A2-2012/175602
- WO-A2-2013/132347
- WO-A2-2014/037588
- LEUKERT N ET AL: "Calcium-dependent Tetramer Formation of S100A8 and S100A9 is Essential for Biological Activity", JOURNAL OF MOLECULAR BIOLOGY, ACADEMIC PRESS, UNITED KINGDOM, vol. 359, no. 4, 16 June 2006 (2006-06-16), pages 961 - 972, XP024951108, ISSN: 0022-2836, [retrieved on 20060616], DOI: 10.1016/J.JMB.2006.04.009
- C. CHAMPAIBOON ET AL: "Calprotectin S100A9 Calcium-binding Loops I and II Are Essential for Keratinocyte Resistance to Bacterial Invasion", JOURNAL OF BIOLOGICAL CHEMISTRY, vol. 284, no. 11, 13 March 2009 (2009-03-13), US, pages 7078 - 7090, XP055261449, ISSN: 0021-9258, DOI: 10.1074/jbc.M806605200
- LUEGERING N ET AL: "Immunohistochemical distribution and serum levels of the Ca2+-binding proteins MRP8, MRP14 and their heterodimeric form MRP8/14 in Crohn's disease", DIGESTION, S. KARGER AG., BASEL, CH, vol. 56, no. 5, 1 January 1995 (1995-01-01), pages 406 - 414, XP009019238, ISSN: 0012-2823, DOI: 10.1159/000201267
- STRIZ I ET AL: "Calprotectin - a pleiotropic molecule in acute and chronic inflammation", PHYSIOLOGICAL RESEARCH, ACADEMIA, PRAGUE, CZ, vol. 53, no. 3, 1 January 2004 (2004-01-01), pages 245 - 253, XP009130467, ISSN: 0862-8408

## Description

### FIELD OF THE INVENTION

The present invention relates to means and methods for detecting calprotectin in a sample as well as compounds for the use therein. The compounds may in particular be mutated calprotectin standards. Provided are assays for the detection of calprotectin for use in the diagnosis of acute and chronic inflammatory diseases.

### BACKGROUND OF THE INVENTION

Acute and chronic inflammations are still a major problem in clinical practice. Especially in chronic cases, the adaptation of the therapy is still the main problem. An over-treatment is associated with the risk of side effects and increased costs, while a sub therapy involves the risk of a new outbreak of inflammation or may be associated with long-term complications of uncontrolled inflammatory response. This problem has increased relevance with the introduction of so-called biologics (usually antibody-based therapies). While it is often possible to achieve remission with these drugs, it is unclear how long and at what dosage the treatment must be continued to treat. Accordingly, this group of drugs has become a large cost factor in our health care system. For these reasons, biomarkers as diagnostic tools that reliably reflect disease progression and detect particular subclinical residual activities of inflammation or even predict relapses are of particular importance. The calcium-binding proteins S100A8 and S100A9 have been proposed as suitable and reliable biomarkers to indicate the course of an inflammatory disease. Both S100 proteins show strong pro-inflammatory activities in many inflammatory reactions, e.g., sepsis, lung and skin infections, arthritis and auto immune diseases. In particular, in rheumatoid arthritis and inflammatory bowel disease in which biologics are often applied for therapy, these biomarkers are clearly superior to conventional laboratory parameters and clinical scores.

S100A8 (myeloid related protein 8, MRP8) and S100A9 (myeloid related protein 14, MRP14) are two members of the low-molecular-weight S100 protein family that exhibit pro-inflammatory activities in many human diseases and play a role in calcium-mediated signaling. Both, S100A8 and S100A9 are usually co-expressed in circulating neutrophils and early differentiation stage of monocytes, as well as in keratinocytes and epithelial cells under inflammatory conditions. During activation of phagocytes, S100A8 and S100A9 are released suggesting intra- as well as extra-cellular functions during inflammation.

As calcium-binding cytosolic molecules S100 proteins are characterized by two calcium-binding EF hands with different affinities for calcium connected by a central hinge region: a high affinity site at the C terminus (EF-hand II) and a low affinity site at the N terminus (EF-hand I). The EF-hand motifs have two a-helices flanking a central calcium-binding loop, thus resulting in a classical helix-loop-helix motif. S100A8 and S100A9 can form monovalent homodimers and a heterodimer known as S100A8/A9 (MRP8/14, calprotectin), as well as even higher oligomeric forms (Hunter and Chazin, J Biol Chem (1998) 273(20): 12427-35, Vogl et al., J Am Soc Mass Spectrom (1999) 10:1124-1130). In this context, distinct hydrophobic amino acids have been identified as directly involved in S100A8/S100A9 dimer formation (Leukert et al., Biol Chem (2005), 386: 429-434). However, simple mixing of both S100A8 and S100A9 subunits is not sufficient for proper heterodimer complex formation, but steps of denaturation/renaturation are necessary for the recombinant complex to show identical properties as S100A8/S100A9 as obtained from granulocytes (Vogl et al., BBA (2006) 1763: 1298-1306, Leukert et al., Biol Chem (2005), 386: 429-434, Foell et al., Clin Chim Acta (2004), 344(1-2): 37-51, Roth et al., Trends Immunol (2003), 24: 383-397.

S100A8 and S100A9 have also been found to oligomerize to (S100A8/S100A9)₂ heterotetramers. Tetramer formation is strictly dependent on the presence of calcium, and in the absence of calcium, heterodimers are the preferred forms of S100A8 and S100A9. The dimer form is known to bind four Ca²⁺-ions, while the (S100A8/S100A9)₂ heterotetramer binds eight Ca²⁺-ions. S100A8 and S100A9 represent the major calcium-binding proteins in phagocytes, and both proteins regulate migration of these cells via modulation of tubulin polymerization. In biological sample, S100A8 and S100A9 generally exist as heterodimers and tetramers.

It has been demonstrated in the art that mutations in EF-hand II do not interfere with formation of S100A8/S100A9 heterodimers, but calcium-induced association to (S100A8/S100A)₂ tetramers is strictly dependent on a functional EF-hand II in S100A9. As shown by Leukert et al., J Mol Biol (2006), 359: 961-972, mutations within the C-terminal EF-hand of S100A9 (e.g. N69A, E79A, N69A+E78A) destroy the high-affinity C-terminal calcium-binding site, leading to failure of tetramer formation in the presence of calcium. This is associated with a lack of functional activity of mutated S100A8/S100A9 complexes in promoting the formation of microtubules. On the other hand, formation of S100A8/S100A9 heterodimers is unaffected by the different mutations in the C-terminal EF-hand.

Lately it has been reported that S100A8/S100A9 function as endogenous TLR4 (Toll-like receptor 4) ligand and due to their specific and high expression at sites of inflammation, one can refer them as a prime candidate for these TLR4-driven sterile inflammatory processes. Accordingly, S100A8/S100A9 heterodimers may be considered as early amplifier of inflammation, inducing pro-inflammatory response in endothelial cells and phagocytes. However, (S100A8/S100A9)₂ tetramerization seems to lead to formation of inactive (S100A8/S100A9)₂ tetramer complexes, which are not able to interact with the TLR4 receptor and thus blocks the S100A8/S100A9 activity and thus the pro-inflammatory, TNFα-releasing pathway (WO2014037588).

Although a number of possible functions for S100A8/S100A9 complex have been proposed, the exact role of these proteins in cell metabolism is still unclear. In human, they have been associated with several inflammatory diseases (Sorg 1992). Phagocytes expressing S100A9 belong to the early infiltrating cells and dominate acute inflammatory lesions. In addition, elevated serum levels of S100A8 and S100A9 have been found in patients suffering from a number of inflammatory disorders including cell arteritis, cystic fibrosis, rheumatoid arthritis, dermatoses, chronic inflammatory bowel disease, chronic bronchitis, some malignancies and autoimmune diseases (Foell et al., Clin Chim Acta (2004), 344(1-2): 37-51, Nacken et al., Microsc Res Tech (2003), 60(6): 569-802003).

As mentioned above, it is important to realize that S100A8 and S100A9 do not occur in isolated forms, but are almost exclusively present as a heterodimeric complex. Furthermore, in the presence of calcium the biologically active S100A8/S100A9 heterodimer oligomerizes to an inactive (S100A8/S100A9)₂ tetramer complex. Accordingly, for the diagnosis and assessment of an inflammatory disease *in vivo* the existing heterodimers seems to be more important than the tetramer forms. However, when using e.g. conventional S100A8/S100A9-ELISAs for quantification of these proteins in biological samples, which are based on diverse antibodies and antibody combinations against one or both proteins, at the moment it cannot be differentiated whether the measured values represent the dimer or tetramer form (WO2013/132347).

Accordingly, there is a need in the art for new means and methods which allow for a differentiation between S100A8/S100A9 heterodimers and tetramers and an accurate detection of S100A8/S100A9 heterodimers in a sample. Such a method would provide a precise diagnostic tool for the detection of a disease associated with S100A8/S100A9 heterodimers. Moreover, such a method would allow for the recognition of the structural state of S100A8/S100A9 proteins in order to reliably reflect the progression of an acute and chronic inflammatory disease. In particular, this method would allow for the exact quantification of the biologic active form of S100A8/S100A9 complex. Moreover, even the detection of subclinical residual activities of inflammation at an early inflammatory state would be possible. The technical problem underlying the present application is thus to comply with this need. The technical problem is solved by providing the embodiments reflected in the claims, described in the description and illustrated in the examples and figures that follow.

### SUMMARY OF THE INVENTION

In the blood of healthy individuals the proteins S100A8 and S100A9 are mainly present in the form of inactive heterotetramers, in the following also referred to as a heterotetrameric complex. However, under inflammatory conditions the S100A8/S100A9 heterodimers, in the following also referred to as a heterodimeric complex, seem to be the more relevant forms of S100A8/S100A9 complexes, having pro-inflammatory effects by interacting with the TLR4 receptor as described in detail in WO2014037588. The present invention discloses for the first time that S100A8/S100A9 heterodimers having at least one mutation in the high- or low-affinity calcium binding region of S100A8 or S100A9 are well suited standards for use in a method of detecting S100A8/S100A9 heterodimers in a sample. This is so, because these S100A8/S100A9 heterodimers are no longer able to tetramerize and thus particularly useful for the reliable evaluation of the amount of S100A8/S100A9 heterodimers in biological samples (e.g. those obtained from the respective patients).

Accordingly, provided herein are methods for detecting, evaluating, diagnosing acute and chronic inflammatory diseases in a vertebrate organism by using the S100A8/S100A9 heterodimer standard of the present invention. In contrast to conventional diagnostic approaches, the method or use as described herein involving the S100A8/S100A9 heterodimer standards of the present invention allow for a reliable detection of heterodimeric S100A8/S100A9 complexes. It is envisaged that such a method or use is more specific (or reliable) than conventional approaches.

In a first aspect the present invention relates to a method of detecting a S100A8/S100A9 heterodimer in a sample, the method comprising the use of an S100A8/S100A9 heterodimer standard which comprises at least one mutation in at least one of the following regions:
a) the high-affinity calcium binding hand of S100A9,
b) the low-affinity calcium binding hand of S100A9,
c) the high-affinity calcium binding hand of S100A8, or
d) the low-affinity calcium binding hand of S100A8.

The S100A8/S100A9 heterodimer standard of the present invention does not tetramerizes to (S100A8/S100A9)₂ tetramers.

The S100A8/S100A9 heterodimer standard may comprise at least one mutation in:
a) the amino acid sequence ranging from amino acid position 63 to amino acid position 79 of the human S100A9 protein of Uniprot/Swissprot accession no. P06702 (SEQ ID NO: 1),
b) the amino acid sequences ranging from amino acid position 20 to amino acid position 38 of the human S100A9 protein of Uniprot/Swissprot accession no. P06702 (SEQ ID NO: 1),
c) the amino acid sequences ranging from amino acid position 55 to amino acid position 71 of the human S100A8 protein of Uniprot/Swissprot accession no. P05109 (SEQ ID NO: 2), or
d) the amino acid sequences ranging from amino acid position 20 to amino acid position 38 of the human S100A8 protein of Uniprot/Swissprot accession no. P05109 (SEQ ID NO: 2), and combinations thereof.

It is also envisaged that the S100A8/S100A9 heterodimer standard comprises at least one mutation selected from:
a) Ser23, Leu26, His28, Thr31, Asp 33, Glu36, Asp67, Asn69, Asp71 or Glu78 of the human S100A9, or
b) Ser23, Lys26, Asn28, Ala31, Asp33, Asp59, Asn61, Asp63 and Glu70 of the human S100A8.
Preferably the at least one mutation is selected from Glu36, Asp67, Asn69, Asp71 and Glu78 of the human S100A9, or from Asp33, Asp59, Asn61, Asp63 and Glu70 of the human S100A8. In some embodiments the mutation comprises the amino acid exchange Asn69Ala or Glu78Ala.

It is envisaged that the detection method is an immunoassay, such as for example a quantitative enzyme-linked immunosorbent assay (ELISA) comprising the following steps:
a) providing a capture antibody capable of binding S100A8 and/or S100A9 (preferably an antibody that captures the S100A8/S100A9 heterodimer specifically),
b) optionally contacting the capture antibody with the sample to be analyzed and the S100A8/S100A9 heterodimer standard as defined herein,
c) optionally washing away unbound sample and standard,
d) optionally contacting bound sample and standard with a detecting antibody,
e) optionally washing away free amounts of the detection antibody,
f) optionally detecting the detection antibody,
g) optionally photometrically determining the absorbance of the sample and the standard, and
h) optionally determining the amount of S100A8/S100A9 heterodimers in the sample by comparing the absorbance with the absorbance of the standard.

It is envisaged that the capture antibody used in the immunoassay, e.g. the quantitative enzyme-linked immunosorbent assay (ELISA), is capable of binding S100A8/S100A9 heterodimers but no S100A8/S100A9 tetramers. In a preferred embodiment the capture antibody capable of binding S100A8/S100A9 heterodimers is an antibody having a binding specificity to an epitope of a vertebrate S100A9 protein, wherein the epitope has an amino acid sequence ranging from (i) amino acid position 63 to amino acid position 79 of the human protein S100A9 of Uniprot/Swissprot accession no. P06702 (SEQ ID NO: 1), or (ii) amino acid position 55 to amino acid position 71 of the human protein S100A8 of Uniprot/Swissprot accession number P05109 (SEQ ID NO: 2).

The quantitative enzyme-linked immunosorbent assay (ELISA) may further comprise the step of comparing the amount of S100A8/S100A9 heterodimer as determined in said assay with the total amount of S100A8/S100A9 protein.

It is envisaged that the sample is a biological sample, such as a stool sample, a serum sample, a plasma sample, an urine sample, a tissue extract sample, a cell or cell culture sample, a sample from a subject suffering from an acute or chronic inflammatory disease, to name some.

The acute or chronic inflammatory disease is in a preferred embodiment rheumatoid arthritis, juvenile idiopathic arthritis, psoriatic arthritis, immune reconstituation inflammatory syndrome (IRIS), sepsis, systemic inflammatory response syndrome (SIRS), pneumonia, osteomyelitis, autoinflammatory syndromes, hyperzincemia, systemic inflammation, atherosclerosis, acute coronary syndrome, myocardial infarction, Crohn's disease, colitis ulcerosa, glomerulonephritis (SLE), diabetes, an inflammatory skin disease, psoriasis, inflammatory bowel disease, vasculitis, allograft rejection, glomerulonephritis, systemic lupus erythematosus, pancreatitis, cancer, dermatomyositis, polymyositis, multiple sclerosis, allergies, autoimmune diseases, cardiovascular diseases, infections (bacterial, viral, by fungi), pulmonary inflammation, systemic onset juvenile idiopathic arthritis (SOJIA), acute lung injury (ALI) and its most severe form, or acute respiratory distress syndrome (ARDS) to name some.

The present invention also provides a diagnostic composition or kit comprising a S100A8/S100A9 heterodimer standard which comprises at least one mutation in at least one of the following regions:
a) the high-affinity calcium binding hand of S100A9,
b) the low-affinity calcium binding hand of S100A9,
c) the high-affinity calcium binding hand of S100A8, or
d) the low-affinity calcium binding hand of S100A8, wherein the S100A8/A9 heterodimer standard does not tetramerize to (S100A8/A9)₂ tetramers.
The kit or diagnostic composition comprising the S100A8/S100A9 heterodimer standard is in a preferred embodiment for use in diagnosis.

The present invention further relates to a S100A8/S100A9 heterodimer standard which comprises at least one mutation in at least one of the following regions:
a) the high-affinity calcium binding hand of S100A9,
b) the low-affinity calcium binding hand of S100A9,
c) the high-affinity calcium binding hand of S100A8, or
d) the low-affinity calcium binding hand of S100A8, wherein the S100A8/A9 heterodimer standard does not tetramerize to (S100A8/A9)₂ tetramers,
or the diagnostic composition or kit comprising the S100A8/S100A9 heterodimer standard for use in a method of diagnosing an acute or chronic inflammatory disease in a human subject.

The diagnosis may comprise the detection of S100A8/S100A9 heterodimer in a biological sample from the human subject.

The present invention also relates to the use of the S100A8/S100A9 heterodimer standard which comprises at least one mutation in at least one of the following regions:
a) the high-affinity calcium binding hand of S100A9,
b) the low-affinity calcium binding hand of S100A9,
c) the high-affinity calcium binding hand of S100A8, or
d) the low-affinity calcium binding hand of S100A8, wherein the S100A8/A9 heterodimer standard does not tetramerize to (S100A8/A9)₂ tetramers,
(in a method of detecting) for detection of S100A8/S100A9 heterodimers in a sample.

The present invention further relates to the use of the S100A8/S100A9 heterodimer standard which comprises at least one mutation in at least one of the following regions:
a) the high-affinity calcium binding hand of S100A9,
b) the low-affinity calcium binding hand of S100A9,
c) the high-affinity calcium binding hand of S100A8, or
d) the low-affinity calcium binding hand of S100A8, wherein the S100A8/A9 heterodimer standard does not tetramerize to (S100A8/A9)₂ tetramers,
in a method of comparing the amount of S100A8/S100A9 heterodimers and tetramers in a sample.

Also disclosed herein is a method of monitoring the progression or defining a specific stage of an acute or chronic inflammatory disease associated with an increased amount of S100A8/S100A9 heterodimer in a patient, the method comprising:
a) quantifying the amount of S100A8/S100A9 heterodimer in a sample (e.g. a sample taken from said patient) by using the S100A8/S100A9 heterodimer standard as defined in claim 1, and
b) comparing the amount of S100A8/S100A9 heterodimer determined in a) with the amount of S100A8/S100A9 heterodimer in a sample from said patient determined at an earlier date, wherein the result of the comparison of b) provides an evaluation of the progression of the inflammatory disease associated with an increased amount of S100A8/S100A9 heterodimer in said patient.
An increased amount of S100A8/S100A9 heterodimer as compared to the reference data indicates a progression of the inflammatory disease associated with an increased amount of S100A8/S100A9 heterodimer in said patient. No change or a decreased amount of S100A8/S100A9 heterodimers as compared to the reference data indicates no progression or a regression of the inflammatory disease associated with an increased amount of S100A8/S100A9 heterodimer in said patient.

The present invention also relates to a method of diagnosing an acute or chronic inflammatory disease in a subject, the method comprising:
a) quantifying the amount of S100A8/S100A9 heterodimer in a sample taken from said subject by using the S100A8/S100A9 heterodimer standard as defined in claim 1, and
b) comparing the amount of S100A8/S100A9 heterodimer as determined in a) to reference data from a subject known to not suffer from an acute or chronic inflammatory disease.
An increased amount of S100A8/S100A9 heterodimer as compared to the reference data indicates that the subject suffers from an acute or chronic inflammatory disease. No significant deviation in the amount of S100A8/S100A9 heterodimer as compared to the reference data indicates that the subject does not suffer from an acute or chronic inflammatory disease.

### DETAILED DESCRIPTION OF THE INVENTION

The following description includes information that may be useful in understanding the present invention. It is not an admission that any of the information provided herein is prior art or relevant to the presently claimed inventions, or that any publication specifically or implicitly referenced is prior art.

The present invention is at least partly based on the surprising finding that S100A8/S100A9 heterodimers comprising at least one mutation in the high- or low-affinity calcium binding hand of S100A8 or in the high- or low-affinity calcium binding hand of S1009 do no longer tetramerize and therefore can be applied as a reliable standard for the detection of S100A8/S100A9 heterodimers in a samples. S100A8/S100A9 heterodimers comprising at least one mutation in one of these regions do not tetramerize to (S100A8/S100A9)₂ tetramer and can thus be considered as a stable S100A8/S100A9 standard.

Accordingly, the S100A8/S100A9 heterodimer standard of the present invention was found to be well suited for standardizing immunoassays (including inter alia established immunoassays that have been used for the detection of S100A8/S100A9 and disease associated therewith, such as acute and chronic inflammatory diseases) and for detecting S100A8/S100A9 heterodimers in a sample. This was unforeseeable since up to now available S100A8/S100A9 detection assays (including the ones that are commercially available) are based on standards comprising an undefined mixture of heterodimeric and heterotetrameric forms, dependent on the calcium concentration in the medium. Accordingly, the methods described in the art are not appropriate to determine the amount of heterodimeric S100A8/S100A9 complexes, but relate to the total amount of S100A8/S100A9 irrespective of the multimerization grade (dimer, tetramer).

As figured out by the present inventors, commercially available quantitative ELISA use capture antibodies which do not differentiate between S100A8/S100A9 heterodimers and tetramers, probably due to the lack of a monoclonal AB which exclusively binds to S100A8/S100A9 heterodimers but not to tetramers. In fact, applying a human S100A8/S100A9 heterodimer standards comprising the S100A9 mutation N69A or E78A in a commercially available S100A8/S100A9 ELISA based on a buffer containing calcium leads to comparable standard values for both the commercial standard and the S100A8/S100A9 heterodimer standard of the present invention. These results indicate that a differentiation between heterodimeric and heterotetrameric complexes is not possible when using commercially available S100A8/S100A9 ELISA. Accordingly, the S100A8/S100A9 heterodimer standard of the present invention may be used to improve and/or standardize the respective immunoassay by providing a defined protein standard.

It is for example envisaged that the standard can be used in methods for generation, selection, and/or maturation etc. of antibodies or similar binding scaffolds, that are specific for the heterodimer S100A8/S100A9 and that are therefore superior to the non-specific antibodies (in terms of the detection of the heterodimer S100A8/S100A9) that are presently employed in commercially available immunoassays. Commercially available assays within the context of the present invention include but are not limited to quantitative S100A8/S100A9 ELISA from e.g. Bühlmann, Aspen Bio Pharma, Hycult, Abnova, Alpco, and Immundiagnostik, to name some. As used herein, a "method for" is equivalent to a "method of" and both terms may be used interchangeably.

The present invention also relates to an antibody whose epitope is comprised either partially or completely in the mutated regions that have been specified herein in the context of the S100A8/S100A9 heterodimer standard. It will be understood that this antibody is specific for the S100A8/S100A9 heterodimer but not specific for the tetramer (i.e. it does not bind to the tetramer). This is so, because its epitope is located in a region that is not accessible for the antibody once the heterodimer tetramerizes. The method of the invention thus comprises the use of an antibody. Antibodies for use herein may be specifically directed against S100A8/S100A9.

The term "antibody" refers to a molecule in which the structure and/or function is/are based on the structure and/or function of an antibody, e.g. of a full-length or whole immunoglobulin molecule. An antibody construct is hence capable of binding to its specific epitope or antigen. Furthermore, an antibody construct according to the invention comprises the minimum structural requirements of an antibody which allow for the epitope binding. This minimum requirement may e.g. be defined by the presence of at least the three light chain CDRs (i.e. CDR1, CDR2 and CDR3 of the VL region) and/or the three heavy chain CDRs (i.e. CDR1, CDR2 and CDR3 of the VH region). The antibodies on which the constructs according to the invention are based include for example monoclonal, recombinant, chimeric, deimmunized, humanized and human antibodies. Within the definition of "antibody" according to the invention are full-length or whole antibodies including camelid antibodies and other immunoglobulin antibodies generated by biotechnological or protein engineering methods or processes. These full length antibodies may be for example monoclonal, recombinant, chimeric, deimmunized, humanized and human antibodies. Also within the definition of "antibody constructs" are fragments of full-length antibodies, such as VH, VHH, VL, (s)dAb, Fv, Fd, Fab, Fab', F(ab')2 or "r IgG" ("half antibody"). Antibodies according to the invention may also be modified fragments of antibodies, also called antibody variants, such as scFv, scFab, Fab2, Fab3, diabodies, single chain diabodies, "minibodies" exemplified by a structure which is as follows: (VH-VL-CH3)2, (scFv-CH3)2 or (scFv-CH3-scFv)2, and single domain antibodies such as nanobodies or single variable domain antibodies comprising merely one variable domain, which might be VHH, VH or VL, that specifically bind an antigen or epitope independently of other V regions or domains.

The methods and assays of the present invention preferably employ the S100A8/S100A9 heterodimer standard and the antibody of the invention.

Moreover, the use of with the S100A8/S100A9 heterodimer standard of the present invention in a detection assay exclusively detecting S100A8/S100A9 heterodimers without detecting S100A8/S100A9 tetramers allows for a specific quantification of S100A8/S100A9 heterodimers in a sample. Accordingly, the S100A8/S100A9 heterodimer standard of the present invention may be employed in a method of diagnosing an inflammatory disease, allowing for the accurate detection of pro-inflammatory heterodimers, which seem to be more relevant for assessing the stage of an inflammatory disease *in vivo.* Thus, the means and methods of the present invention allow for detecting, diagnosing, monitoring etc. an acute and chronic inflammatory disease.

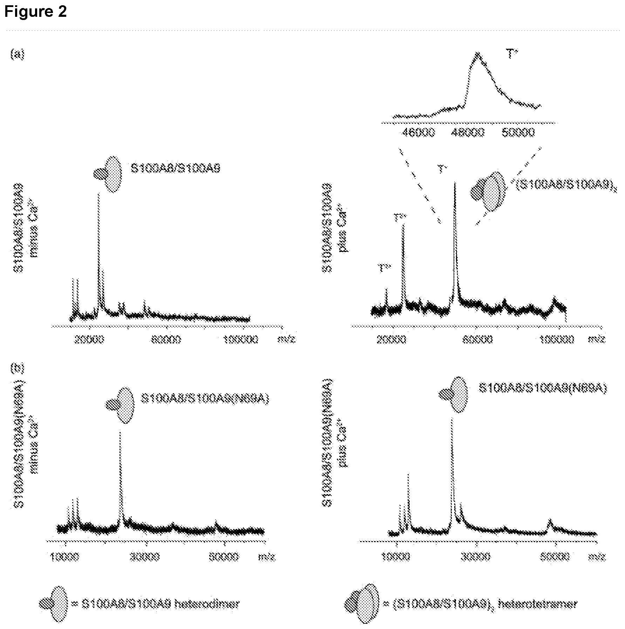

S100A8/S100A9 heterodimers having a mutation in the high-affinity hand of S100A9 at amino acid position 69 or 78 of the human S100A9 protein of Uniprot/Swissprot accession no. P06702 (version 1 as of 1 January 1988, SEQ ID NO: 1) do not tetramerize to (S100A8/S100A9)₂ tetramers (Figures 2, 3, and 5). The use of said heterodimers as standard in a method of detecting S100A8/S100A9 in a sample has never been described before. Also the standardization of such detecting methods by using mutated S100A8/S100A9 heterodimer standards has not been disclosed so far. The inventors of the present application further defined additional amino acid position in the high- and low-affinity calcium binding hands of S100A8 and S100A9 which seem to be particularly relevant for the calcium binding. S100A8/S100A9 heterodimers having a mutation in at least one of the identified positions lack essential ligands for calcium-coordination. While the dimerization is unaffected by said mutations, the mutated heterodimers of the present invention do not significantly tetramerize and/or polymerize to higher oligomers.

It is envisaged that the S100A8/S100A9 heterodimer standard does not significantly tetramerize but is still capable of forming heterodimers (S100A8/S100A9).

In one aspect, the present invention relates to a method of detecting a S100A8/S100A9 heterodimer in a sample, the method comprising the use of an S100A8/S100A9 heterodimer standard. The term "S100A8/S100A9 heterodimer standard" when used herein relates to a S100A8/S100A9 heterodimer comprising at least one mutation in the high-affinity calcium binding hand of S100A9, the low-affinity calcium binding hand of S100A9, the high-affinity calcium binding hand of S100A8, or the low-affinity calcium binding hand of S100A8. The term "at least one mutation" when used herein means that at least one of the naturally occurring amino acid in any of the position in the defined region is exchanged by another amino acid not naturally occurring in said position. It is envisaged that the amino acid which replaces the naturally occurring amino acid at the respective position is replaced/exchanged by an amino acid having comparable physicochemical characteristics. It is also envisaged that the replacing amino acid is characterized by the same side chain group type (e.g. aliphatic, acyclic, aromatic etc.). In the context of the present invention it is further envisaged that more than one position is mutated. Accordingly, the present invention also relates to a S100A8/S100A9 heterodimer standard comprising at least 2, 3, 4, 5, 6, 7, 8, 9, or 10 or more mutations in the low- or high-affinity calcium binding hands of S100A8 and/or S100A9 as explained herein. The amino acid exchange leads in a preferred embodiment to a reduced affinity to calcium as compared to a S100A8/S100A9 heterodimer which does not comprise a mutation in any of the defined regions. It will be understood that the S100A8/S100A9 heterodimer standard of the present invention does not tetramerize to (S100A8/S100A9) tetramers, but exclusively exists as a heterodimeric complex. This is so, because the mutation prevents the oligomerization of the heterodimer to a tetramer (i.e. it does not significantly tetramerize). The term "tetramerize" means that two S100A8/S100A9 heterodimers oligomerize to a tetramer in a calcium-dependent manner. "Significantly" means that a S100A8/S100A9 heterodimer standard may still comprise up to 10%, preferably up to 5%, more preferably up to just 1% , even more preferably up to just 0,5% (w/w) impurity of S100A8/S100A9 tetramers when compared to the total amount of S100A8 and S100A9. The standard comprises in a most preferred embodiment no detectable impurity with S100A8/S100A9 tetramers.

The inventors of the present application further defined specific amino acid regions in the human S100A8 and S100A9 protein which seem to be particularly relevant for the calcium binding. In fact, human S100A8/S100A9 heterodimers having a mutation in at least one of the identified regions lack essential amino acids for the calcium-coordination. In some embodiments the S100A8/S100A9 heterodimer standard comprises at least one mutation in amino acid sequence ranging from amino acid position 63 to amino acid position 79 of the human S100A9 protein of Uniprot/Swissprot accession no. P06702 (version 1 as of 1 January 1988, SEQ ID NO: 1), the amino acid sequences ranging from amino acid position 20 to amino acid position 38 of the human S100A9 protein of Uniprot/Swissprot accession no. P06702 (version 1 as of 1 January 1988, SEQ ID NO: 1), the amino acid sequences ranging from amino acid position 55 to amino acid position 71 of the human S100A8 protein of Uniprot/Swissprot accession no. P05109 (version 1 as of 1 January 1988, SEQ ID NO: 2), or the amino acid sequences ranging from amino acid position 20 to amino acid position 38 of the human S100A8 protein of Uniprot/Swissprot accession no. P05109 (version 1 as of 1 January 1988, SEQ ID NO: 2). Preferably, the S100A8/S100A9 heterodimer standard comprises at least one mutation selected from Ser23, Leu26, His28, Thr31, Asp 33, Glu36, Asp67, Asn69, Asp71 or Glu78 of human S100A9, or Ser23, Lys26, Asn28, Ala31, Asp33, Asp59, Asn61, Asp63 and Glu70 of human S100A8. Preferably the S100A8/S100A9 heterodimer standard comprises at least one mutation selected from Glu36, Asp67, Asn69, Asp71 and Glu78 of human S100A9 or Asp33, Asp59, Asn61, Asp63 and Glu70 of human S100A8. In one embodiment the mutation comprises the amino acid exchange Asn69Ala or Glu78Ala in human S100A9 protein.
SEQ ID NO: 1 (human S100A9 protein) 
MTCKMSQLERNIETIINTFHQYSVKLGHPDTLNQGEFKELVRKDLQNFLK
KENKNEKVIEHIMEDLDTNADKQLSFEEFIMLMARLTWASHEKMHEGDEG
PGHHHKPGLG EGTP
SEQ ID NO: 2 (human S100A8 protein) 
MLTELEKALNSIIDVYHKYSLIKGNFHAVYRDDLKKLLETECPQYIRKKG
ADVWFKELDI NTDGAVNFQE FLILVIKMGV AAHKKSHEES HKE
SEQ ID NO: 3 (murine S100A9 protein) 
MANKAPSQMERSITTIIDTFHQYSRKEGHPDTLSKKEFRQMVEAQLATFM
KKEKRNEALINDIMEDLDTNQDNQLSFEECMMLMAKLIFACHEKLHENNP
RGHGHSHGKG CGK
SEQ ID NO: 4 (murine S100A8 protein)

MPSELEKALSNLIDVYHNYSNIQGNHHALYKNDFKKMVTTECPQFVQNIN
IENLFRELDI NSDNAINFEE FLAMVIKVGV ASHKDSHKE

It is also envisaged that the standard comprises mutations such as amino acid exchanges outside the mutated regions and/or modifications that are located within and/or outside the mutated regions that have been defined herein, as long as these further mutations/modifications do not significantly alter the S100A8/S100A9 heterodimer structure. Thus, modifications and/or further alterations (e.g. truncations) are allowed as long as these modifications/alterations do not affect the heterodimer. It is particularly envisaged that the alterations and/or modifications do not affect the binding specificity of the respective antibody (i.e once an antibody that is specific for the heterodimer is chosen, it is envisaged that the heterodimer standard can be modified but this modification or alteration should preferably not affect the epitope of this respective antibody).

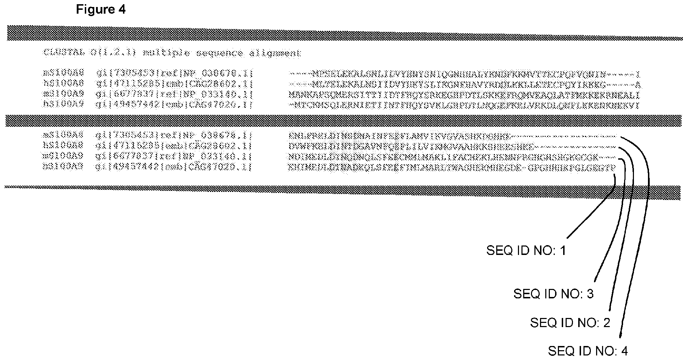

As described for human S100A8/S100A9 heterodimer standards, the system characterizing the S100A8/S100A9 heterodimer standard of the present invention can be easily transferred by the person skilled in the art to other mammalian species in order to identify homolog positions. As shown in Figure 4 for murine S100A8 and S100A9, the low- and high-affinity calcium binding regions and positions corresponding to the human S100A8/S100A9 heterodimer standard can be easily identified by aligning the respective S100A8 and S100A9 amino acid sequences. Those skilled in the art will be familiar with the fact that corresponding sequences need to be compared. The use of a corresponding sequence includes that a position is not only determined by the number of the preceding amino acids. Accordingly, the position of a given amino acid in accordance with the disclosure which may be substituted may very due to deletion or addition of amino acids elsewhere in the protein such as the S100A8 or S100A9 protein. Thus, by a "corresponding position" in accordance with the disclosure it is to be understood that amino acids may differ in the indicated number - for instance when comparing data base entries - but may still have similar neighbouring amino acids. Such amino acids which may be exchanged, deleted or added are also included in the term "corresponding position".

Accordingly, in some embodiments the S100A8/S100A9 heterodimer standards of the present invention comprise at least one mutation selected from Ser23, Glu26, His28, Thr31, Asp33, Glu37, Asp68, Asn70, Asp72 and Glu79 of the murine S100A9 protein of Uniprot/Swissprot accession no. P31725 (version 3 as of 23 January 2007, SEQ ID NO: 3), or Ser23, Gln26, Asn28, Ala31, Asp33, Asp59, Asn61, Asp63 and Glu70 of the murine S100A8 protein of Uniprot/Swissprot accession no. P27005 (version 3 as of 23 January 2007, SEQ ID NO: 4). Preferably the S100A8/S100A9 heterodimer standard comprises at least one mutation selected from Glu37, Asp68, Asn70, Asp72 and Glu79 of murine S100A9 or Asp33, Asp59, Asn61, Asp63 and Glu70 of the murine S100A8.

The term "position" when used in accordance with this disclosure means the position of an amino acid within an amino acid sequence depicted herein.

Also all combinations of the mutations described herein for the different species are within the scope of the present invention. Accordingly, combinations comprising several mutations in each of the high- or low-affinity calcium binding regions, as well as combination comprising just one mutation in each of the high- or low-affinity calcium binding regions are comprised by the present invention.

The term "detecting" when used herein includes variations like determining, qualifying, semi-qualifying or, as the case may be, diagnosing etc. The term "detect" or "detecting", as well as the term "determine" or "determining" when used in the context of a biomarker refers to any method that can be used to identify the presence of a protein/polypeptide released or expressed by a cell. In some embodiments the method of detecting a S100A8/S100A9 heterodimer in a sample may be a method of detecting the level (quantitative or semi-quantitative) of S100A8/S100A9, by comparing the level of S100A8/S100A9 heterodimer in the sample with the level of S100A8/S100A9 heterodimer standard. The methods of the present invention are preferably *in vitro* methods.

As described above, the present invention relates to the use of the S100A8/S100A9 heterodimer standard as defined herein in a method of detecting S100A8/S100A9 heterodimers in a sample. In this context, it is also envisaged to use the S100A8/S100A9 heterodimer standard for the standardization of established detection methods which do not differentiate between S100A8/S100A9 heterodimer and tetramers, but detect the total amount of S100A8/S100A9 in a sample. In this regard established detection methods can also be compared using on the one hand the commercial standards and on the other hand the S100A8/S100A9 heterodimer standard of the present invention. Furthermore, S100A8/S100A9 heterodimer standard can be used in displacement assays. In some embodiments the detecting methods are quantitative immunoassays such as quantitative enzyme-linked immunosorbent assays (ELISAs). Accordingly, the present invention also provides for a method of standardizing a quantitative S100A8/S100A9 immunoassay by using the S100A8/S100A9 heterodimer standard of the present invention as protein standard in said immunoassay.

In a preferred embodiment the quantitative ELISA is a quantitative sandwich ELISA comprising the following steps:
a) pre-coating a microplate with a monoclonal capture antibody capable of binding S100A8 and/or S100A9,
b) optionally contacting the pre-coated capture antibody with the sample to be analyzed and the standard as defined in claim 1,
c) optionally washing away unbound sample and standard,
d) optionally contacting bound sample and standard with an enzyme-conjugated detecting antibody,
e) optionally washing away free amounts of the detecting antibody,
f) optionally contacting the bond detecting antibody with the substrate of the conjugated enzyme,
g) optionally finishing the enzymatic reaction,
h) optionally photometrically determining the absorbance of the sample and the standard, and
a) optionally determining the amount of S100A8/S100A9 heterodimer in the sample by comparing the absorbance with the absorbance of the standard.

The term "sample" when used herein relates to a material or mixture of materials, typically but not necessarily in liquid form, containing one or more analytes of interest. Preferably, the sample of the present invention is a biological sample. The term "biological sample", as used herein, refers to a sample obtained from an organism or from components (e.g., cells) of an organism. The sample may be of any biological tissue or fluid. Frequently the sample will be a "clinical sample" which is a sample derived from a patient. The samples described herein include but are not limited to a stool sample, a blood sample, a serum sample, a plasma sample, an urine sample, a tissues extract sample or a cell culture sample, a stool sample, plasma sample or a serum sample being preferred. Biological samples from a subject may be obtained by an individual undergoing a self-diagnostic test (e.g., blood glucose monitoring) or by a trained medical professional through a variety of techniques including, for example, aspirating blood using a needle or scraping or swabbing a particular area. Methods for collecting various biological samples are well known in the art.

The term "subject" as used herein, also addressed as an individual, refers to a mammalian organism including a human or a non-human animal. Thus, the methods, uses and compositions described in this document are generally applicable to both human and animals. As explained above, a sample may be analyzed that has been obtained from said subject, which is typically a living organism. Where the subject is a living human who may receive treatment or diagnosis for a disease or condition as described herein, it is also addressed as a "patient".

The S100A8/S100A9 heterodimers have been proposed as suitable and reliable biomarkers to indicate the course of an inflammatory disease. The term "biomarker" is defined as a physical sign or laboratory measurement that occurs in association with a natural or pathological process, and that has putative diagnostic and/or prognostic utility. More precisely, the term "biomarker" may comprise a protein or a gene encoding a protein/peptide, which is expressed at a lower or higher level by a cell under different cellular conditions. In the present disclosure, said biomarker is preferably expressed and released by a subject under native and/or pathological conditions, such as inflammatory conditions. The biomarker described herein is usually expressed and released by an immune cell, in particular neutrophils, early differentiation stage of monocytes, keratinocytes and epithelial cells. Said biomarker is preferably S100A8/S100A9 which generally exists as heterodimer or heterotetramer depending on the amount of calcium at the release site. In the present invention, measuring the level of the heterodimeric complex of said biomarkers can be used for diagnosing and/or monitoring an acute or chronic inflammatory disease.

In the context of the present invention, the sample as described in the methods and uses herein is a sample from a subject suffering from an acute or chronic inflammatory disease. The acute or chronic inflammatory disease is essentially an inflammatory disease associated with an increased amount of S100A8/S100A9 heterodimer. This disease is selected from rheumatoid arthritis, juvenile idiopathic arthritis, psoriatic arthritis, immune reconstituation inflammatory syndrome (IRIS), sepsis, systemic inflammatory response syndrome (SIRS), pneumonia, osteomyelitis, autoinflammatory syndromes, hyperzincemia, systemic inflammation, atherosclerosis, acute coronary syndrome, myocardial infarction, Crohn's disease, colitis ulcerosa, glomerulonephritis (SLE), diabetes, an inflammatory skin disease, psoriasis, inflammatory bowel disease, vasculitis, allograft rejection, glomerulonephritis, systemic lupus erythematosus, pancreatitis, a cancer, dermatomyositis and polymyositis, multiple sclerosis, allergies, autoimmune diseases, cardiovascular diseases, infections, pulmonary inflammation, systemic onset juvenile idiopathic arthritis (SOJIA), acute lung injury (ALI) and its most severe form, acute respiratory distress syndrome (ARDS).

As described above, commercially available S100A8/S100A9 ELISAs do not differentiate between S100A8/S100A9 heterodimers and the tetramer, because the capture antibody used in these assays equally binds to both forms. Moreover, these assays miss a defined protein standard, and the heterodimer-tetramer ratio is strictly dependent on the amount of calcium in the buffer. Thus, the S100A8/S100A9 heterodimer standards of the present invention may be well suited to standardize these commercially available S100A8/S100A9 ELISAs by provided a defined protein standard. Accordingly, the present invention also provides for a method of detecting S100A8/S100A9 in a biological sample, the method comprising the use of at least one mutation in at least one of the following regions:
b) the high-affinity calcium binding hand of S100A9,
c) the low-affinity calcium binding hand of S100A9,
d) the high-affinity calcium binding hand of S100A8, or
e) the low-affinity calcium binding hand of S100A8.

According to the present invention, the S100A8/S100A9 heterodimers as defined herein may further be used in a method of detecting S100A8/S100A9 heterodimers in a sample. These methods are based on a distinction between the heterodimeric and tetrameric S100A8/S100A9 complex. In this context, monoclonal capture antibodies are used which are capable of binding S100A8/S100A9 heterodimers but no S100A8/S100A9 tetramers. This necessarily implies that the antibody has a binding specificity to an epitope which is freely accessible in the S100A8/S100A9 heterodimer, but not available in the tetrameric form. A variety of antibodies capable of binding S100A8/S100A9 heterodimers but no tetramers are described in WO2014037588 and are within the scope of the present invention. As an illustrative example, the capture antibody may be an antibody having a binding specificity to an epitope of a vertebrate S100A9 protein, wherein the epitope has an amino acid sequence ranging from (i) amino acid position 63 to amino acid position 79 of the human protein S100A9 of Uniprot/Swissprot accession no. P06702 (version 1 as of 1 January 1988, SEQ ID NO: 1), or (ii) amino acid position 55 to amino acid position 71 of the human protein S100A8 of Uniprot/Swissprot accession number P05109 (version 1 as of 1 January 1988, SEQ ID NO: 2).

In some embodiments the method of quantifying S100A8/S100A9 heterodimers further comprises the step of comparing the amount of S100A8/S100A9 heterodimers with the total amount of S100A8/S100A9. The term "total amount" as used herein represents the sum of S100A8/S100A9 heterodimers and tetramers in a sample to be analyzed. This comparison will be well suited to determine the amount of S100A8/S100A9 tetramers in a sample and to assess the heterodimer-tetramer-ratio. Accordingly, the present invention also relates to the use of the S100A8/S100A9 heterodimer standard as defined above in a method of comparing the amount of S100A8/S100A9 heterodimers and tetramers in a sample. Since in healthy subjects the proteins S100A8 and S100A9 are mainly present in the form of inactive heterotetramers, the heterodimer-tetramer-ratio will be appropriate for monitoring the progression or regression of an inflammatory disease associated with an increased amount of S100A8/S100A9 heterodimer. A shift towards the S100A8/S100A9 heterodimer may indicate the progression of an inflammatory disease, whereas a shift towards the S100A8/S100A9 tetramer may indicate a regression of an inflammatory disease. The total amount of S100A8/S100A9 can be determined by any of the commercially available ELISAs described elsewhere herein or by an alternative method. The skilled artisan is aware of the variety of techniques used for protein quantification such as photometric or chromatographic methods.

Alternatively the amount of S100A8/S100A9 tetramer can be quantified using FRET-based technology. This technic requires the use of two S100A8 or two S100A9 fluorecence-labelled antibodies, wherein only the detection of a tetramer triggers the corresponding FRET-signal. Accordingly, also disclosed herein is a method of quantifying S100A8/S100A9 in a sample, the method comprising quantifying the amount of S100A8/S100A9 tetramers. This method can be used to evaluate the results of commercially available S100A8/S100A9 assays.

In a further aspect, the present invention provides a kit comprising the S100A8/S100A9 heterodimer standard of the present invention. The kit is preferably for us in diagnosis. The diagnostic kit of the invention comprising a binding molecule which specifically binds to S100A8/S100A9 is preferably carried out by an ELISA or immunochromatographic technique. According to some embodiments, the term "kit" when used herein refers to an assembly of useful compounds and other means like solid support plates or test stripes for detecting S100A8/S100A9 in a mammalian sample. A kit therefore may include the standard and/or composition of the present invention. In one particular example, such a kit includes a device having an immobilized capture antibody and other useful reagents like wash reagent, as well as detector reagent and the S100A8/S100A9 heterodimer standard of the present invention. Other components such as buffers, controls, and the like, known to those skilled in art, may be included in such test kits. The relative amounts of the various reagents can be varied, to provide for concentrations in solution of the reagents that substantially optimize the sensitivity of the assay. Particularly, the reagents can be provided as dry powders, usually lyophilized, which on dissolution will provide for a reagent solution having the appropriate concentrations for combining with a sample. The present kit may further include instructions for carrying out one or more methods of the present invention, including instructions for using standard and/or composition of the present invention that is included with the kit. In some embodiments the diagnostic kit comprises a monoclonal antibody binding to S100A8/A9 heterodimers and tetramers. In some embodiments the diagnostic kit comprises a monoclonal antibody exclusively binding to S100A8/S100A9 heterodimers but not to tetramers. The antibodies used in said kit can be present in bound or soluble form. In some embodiments the monoclonal antibody is 27E10. In some embodiments the monoclonal antibody is any of the antibodies described in WO2014037588.

Another aspect disclosed herein related to a diagnostic composition comprising S100A8/S100A9 heterodimer standard of the present invention. In some embodiments the diagnostic composition comprises a mixture of a binding molecule which specifically binds to S100A8/S100A9 and chemical reagents. The binding molecule is preferably a monoclonal antibody binding to S100A8/A9 heterodimers and tetramers, or a monoclonal antibody exclusively binding to S100A8/S100A9 heterodimers. The antibody is present in soluble form. In some embodiments the monoclonal antibody is 27E10. In some embodiments the monoclonal antibody is any of the antibodies described in WO2014037588.

In the context of the present invention, the S100A8/S100A9 heterodimer standard, the kit comprising said standard or the diagnostic composition comprising said standard may be used in a method of diagnosing an acute or chronic inflammatory disease in a human subject. Accordingly, a method of diagnosing an acute or chronic inflammatory disease in a subject is also provided herein. In this context the S100A8/S100A9 heterodimer standard, the kit comprising said standard or the diagnostic composition comprising said standard are preferably used for diagnosing whether a subject is suffering from any of the acute or chronic inflammatory disease described elsewhere herein. Accordingly, a sample takes from said subject is analyzed using the S100A8/S100A9 heterodimer standard, the kit comprising said standard or the diagnostic composition comprising said standard to the present invention. The sample can be any of the samples as described herein above.

In some embodiments the method of diagnosing an acute or chronic inflammatory disease in a subject comprises a) quantifying the amount of S100A8/S100A9 heterodimer in a sample taken from said subject by using the S100A8/S100A9 heterodimer standard of the present invention and b) comparing the amount of S100A8/S100A9 heterodimer as determined in a) to reference data from a subject known to not suffer from an acute or chronic inflammatory disease. A significant increased amount of S100A8/S100A9 heterodimer as compared to the reference data indicates that the subject suffers from an acute or chronic inflammatory disease. No significant deviation in the amount of S100A8/S100A9 heterodimer as compared to the reference data indicates that the subject does not suffer from an acute or chronic inflammatory disease.

Moreover, detecting the amount of S100A8/S100A9 heterodimer in a sample by using the S100A8/S100A9 heterodimer standard of the present invention can be used to define or evaluate the specific state of an acute or chronic inflammatory disease. Depending on the level of pro-inflammatory S100A8/S100A9 heterodimer in a subject, the disease can be defined or evaluated as to be in a state of progression or remission. Moreover, also the risk of relapse during remission can be predicted or the efficacy of novel therapeutics in treatment of inflammatory diseases such as antibody-based therapies can be evaluated. Evaluating the efficacy of anti-inflammatory compounds refers to the assessment of whether or not a patient suffering from an inflammatory disease is responsive to said therapeutic compound. In this context, the amount of S100A8/S100A9 in a sample from a subject will be predictive to assess if the therapy is effective or not. Thus, the amount of S100A8/S100A9 heterodimer in a sample from said patient has to be compared with the amount of S100A8/S100A9 heterodimer in a sample from said patient determined at an earlier date, preferably before initiation of treatment. A significantly increased amount of S100A8/S100A9 heterodimer as compared to the reference data indicates a progression of the inflammatory disease associated with an increased amount of S100A8/S100A9 heterodimer in said patient. No change or a decreased amount of S100A8/S100A9 heterodimer as compared to the reference data indicates no progression or a regression of the inflammatory disease associated with an increased amount of S100A8/S100A9 heterodimer in said patient. This principle is equally applicable for the evaluation of the progression of an inflammatory disease. Accordingly, the present invention further relates to a method of evaluating the progression of an inflammatory disease in a patient suffering from an acute or chronic inflammatory disease. Such evaluation may help an attending physician to obtain the appropriate information to set the appropriate therapy conditions for the treatment of the respective inflammatory disease. Also a method for evaluating whether a subject may be of a risk to develop an inflammatory disease is comprised by the present invention.

Additionally, the progression of an acute or chronic inflammatory disease in a patient can be monitored. Monitoring the progression indicates that the amount of S100A8/S100A9 heterodimers in said patient is over a certain period regularly or occasionally determined. The method for monitoring the progression of a disease in a patient can be a short-term monitoring or a long-term monitoring. Preferably, the amount of S100A8/S100A9 heterodimer in a sample taken from said patient is quantified by any of the methods described herein. The amount of S100A8/S100A9 heterodimer is further compared with a reference sample from said patient quantified at an earlier date, wherein the result of the comparison provides an evaluation of the progression of the inflammatory disease. A significantly increased amount of S100A8/S100A9 heterodimer as compared to the reference data indicates a progression of the inflammatory disease, wherein no change or a decreased amount of S100A8/S100A9 heterodimer as compared to the reference data indicates no progression or a regression of the inflammatory disease.

Unless otherwise stated, the following terms used in this document, including the description and claims, have the definitions given below.

Those skilled in the art will recognize, or be able to ascertain, using not more than routine experimentation, many equivalents to the specific embodiments of the invention described herein.

It is to be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the methods and uses described herein.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "consisting", "consisting of" and "consisting essentially of" may be replaced with either of the other two terms.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

As described herein, "preferred embodiment" means "preferred embodiment of the present invention". Likewise, as described herein, "various embodiments" and "another embodiment" means "various embodiments of the present invention" and "another embodiment of the present invention".

The word "about" as used herein refers to a value being within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects according to the invention. In this context "about" may refer to a range above and/or below of up to 10%. The word "about" refers in some embodiments to a range above and below a certain value that is up to 5%, such as up to up to 2%, up to 1%, or up to 0.5 % above or below that value. In one embodiment "about" refers to a range up to 0.1 % above and below a given value.

### BRIEF DESCRIPTION OF THE DRAWINGS

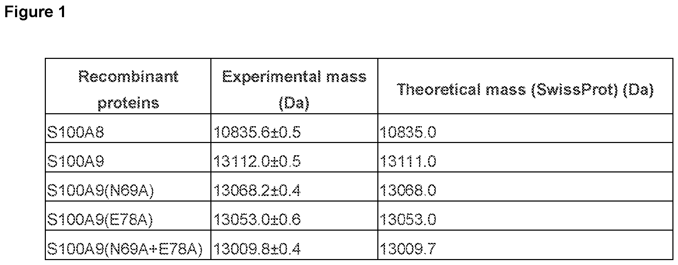

**Figure 1****: Molecular masses determined by ESI-MS.** The molecular masses of the recombinant S100A8 and S100A9 proteins are determined by ESI-MS under denaturing conditions and compared with their theoretical calculated masses (SwissProt, www.expasy.org).
**Figure 2****: MALDI mass spectra in the absence and presence of calcium under** native **solvent conditions.** (a) MALDI mass spectra of recS100A8/S100A9 wt complexes in the absence (left) and presence (right) of calcium using 2,6-dihydroxy-acetophenone as matrix. The mass spectra between *m*/*z* 20,000 and 100,000 together with an inset between *m*/*z* 46,000 and 50,000 are shown. T⁺, singly charged; T²⁺, doubly charged; T³⁺, triply charged tetramer. (b) MALDI mass spectra of recS100A8/S100A9(N69A) mutant complexes in the absence (left) and presence (right) of calcium. RecS100A8/S100A9(E78A) and recS100A8/S100A9(N69A+E78A) showed almost identical results (data not shown).

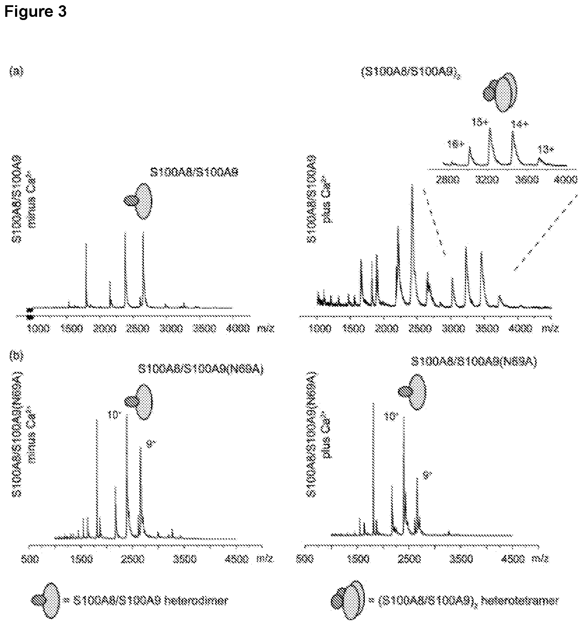

**Figure 3****: ESI mass spectra in the absence and presence of calcium under native solvent conditions.** (a) ESI mass spectra of recS100A8/S100A9 wild-type complexes. The mass spectra between *m*/*z* 1000 and 4000 together with an inset between *m*/*z* 2800 and 4000 are shown. In the absence of calcium (left) the main signals observed correspond to heterodimers with charge states 10+ and 9+. In the presence of calcium (right) signals corresponding to tetramers occurred at charge states 16+, 15+, 14+ and 13+ in the range between *m*/*z* 3000-3800. (b) ESI mass spectra of recS100A8/S100A9(N69A) mutant complexes. In the absence and presence of calcium the main signals correspond to heterodimers with charge states 10+ and 9+, no tetramers were found. recS100A8/S100A9(E78A) and recS100A8/S100A9(N69A+E78A) showed almost identical results (data not shown).
**Figure 4****: Multiple sequence alignment of human and murine S100A8 and S100A9 protein sequences.** The upper illustration represents a partial sequence comprising the low-affinity calcium binding hand of human and murine S100A8 and S100A9. The lower illustration represents a partial sequence comprising the high-affinity calcium binding hand of human and murine S100A8 and S100A9. Some of the corresponding positions relevant for the calcium binding in human and murine S100A8/S100A9 are shaded in grey.

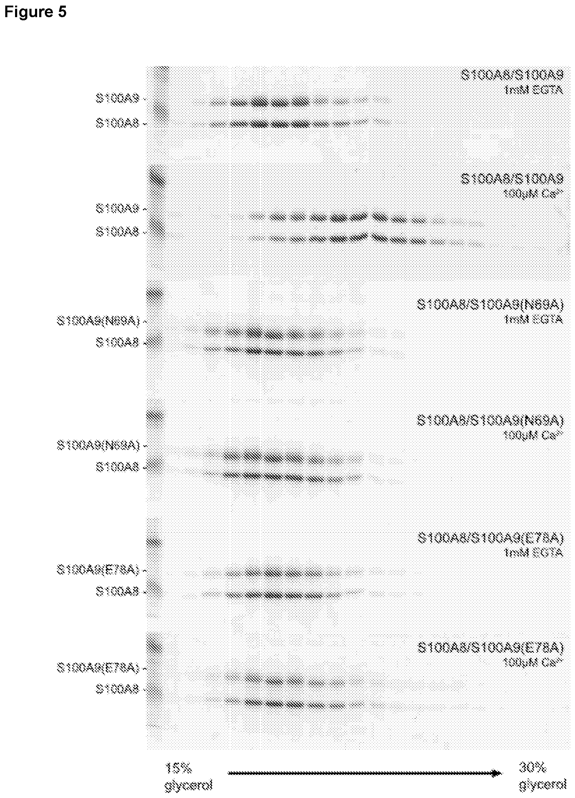

**Figure 5****: Density gradient centrifugation.** wt and mutant recS100A8/S100A9 complexes were loaded on a glycerol gradient in the presence of either 1 mM EGTA or 100 µM Ca²⁺. After centrifugation, successive fractions of the gradients were analyzed by SDS-PAGE. In the presence of EGTA wt and mutant complexes showed an almost identical distribution centered in the low density fractions of the gradient. Addition of calcium induced a marked shift for recS100A8/S100A9 wt to higher glycerol densities, whereas for the mutant complexes recS100A8/S100A9(N69A) and recS100A8/S100A9(E78A) no shift was observed.

### EXAMPLES

The following examples illustrate the invention. These examples should not be construed as to limit the scope of this invention. The examples are included for purposes of illustration and the present invention is limited only by the claims.

### Differentiation between S100A8/S100A9 heterodimers and teteramers using MALDI-MS and ESI-MS

Electrospray ionization mass spectrometry (ESI-MS) confirms the theoretically calculated masses without the N-terminal methionine (SwissProt) for mutated and non-mutated recombinant S100A8/S1009 proteins (Figure 1).

S100A8 and S100A9 exist as heterodimers in the absence of calcium, and these heterodimeric complexes associate to (S100A8/S100A9)₂ tetramers upon calcium-binding. Figure 2(a) shows the matrix-assisted laser desorption/ionisation mass spectrometry (MALDI-MS) spectra of the recS100A8/S100A9 wt proteins. In the absence of calcium, samples show intense signals of singly charged heterodimers. In contrast, in the presence of Ca²⁺ wt S100A8/S100A9 show a base peak in first shot spectra that corresponds to a singly charged heterotetramer (T⁺: 48 kDa) composed of two molecules recS100A8 and two molecules of recS100A9, respectively. Other prominent signals are detected at molecular masses of around 24 kDa and 16 kDa, representing doubly charged (T²⁺) or triply charged (T³⁺) tetramers in accordance with results reported earlier for the native proteins purified from human granulocytes. The number of Ca²⁺ bound to the tetramers was calculated from the difference between the observed masses of the tetramers and the sum of the calculated theoretical molecular masses of the monomeric components.

The oligomerization properties of the recS100A8/S100A9 mutant complexes can be determined by MALDI-MS. As shown exemplarily for the N69A mutant in Figure 2(b), all mutant S100A9 proteins display signals for singly charged heterodimers in the absence of calcium. In contrast to the results obtained with the recS100A8/S100A9 wt proteins no heterotetramers can be observed for the mutant complexes in the presence of calcium (Figure 2). The base peaks under these conditions exclusively represent S100A8/S100A9(N69A), S100A8/S100A9(E78A) and S100A8/S100A9(N69A+E78A) heterodimers. All MALDI-MS experiments presented here were also confirmed by ESI-MS measurements (see Figure 3).

Density gradient centrifugation can be employed in order to confirm the different complex formation patterns obtained in the mass spectrometric studies (Figure 5). In EGTA-containing samples the recS100A8/S100A9 wt and mutant complexes are found in the same range of fractions of the glycerol gradient (19(±2)%), indicating that in the absence of calcium the formation of heterodimers is preferred in wt proteins and all S100A9 mutants. In the presence of calcium, wt complexes shifted to fractions of significantly higher glycerol concentrations (23(±2)%), as observed earlier for S100A8/S100A9 purified from granulocytes. This shift reflects the calcium-induced formation of high-molecular (S100A8/S100A9)₂ tetramers. In contrast, after addition of calcium, the mutant complexes recS100A8/S100A9(N69A) and recS100A8/S100A9(E78A) show no shift to higher glycerol concentrations, confirming that heterotetramer formation is disturbed.

## Claims

1. A method for detecting a S100A8/S100A9 heterodimer in a biological sample, the method comprising the use of an S100A8/S100A9 heterodimer as a standard which comprises at least one mutation in at least one of the following regions:
a) the high-affinity calcium binding hand of S100A9,
b) the low-affinity calcium binding hand of S100A9,
c) the high-affinity calcium binding hand of S100A8, or
d) the low-affinity calcium binding hand of S100A8,
wherein the S100A8/S100A9 heterodimer standard does not tetramerize to (S100A8/S100A9)₂ tetramers.

2. The method of claim 1, wherein the S100A8/S100A9 heterodimer standard comprises at least one mutation in:
a) the amino acid sequence ranging from amino acid position 63 to amino acid position 79 of the human S100A9 protein of Uniprot/Swissprot accession no. P06702 (SEQ ID NO: 1),
b) the amino acid sequences ranging from amino acid position 20 to amino acid position 38 of the human S100A9 protein of Uniprot/Swissprot accession no. P06702 (SEQ ID NO: 1),
c) the amino acid sequences ranging from amino acid position 55 to amino acid position 71 of the human S100A8 protein of Uniprot/Swissprot accession no. P05109 (SEQ ID NO: 2), or
d) the amino acid sequences ranging from amino acid position 20 to amino acid position 38 of the human S100A8 protein of Uniprot/Swissprot accession no. P05109 (SEQ ID NO: 2), and combinations thereof,
wherein the S100A8/S100A9 heterodimer standard preferably comprises at least one mutation selected from:
i) Ser23, Leu26, His28, Thr31, Asp 33, Glu36, Asp67, Asn69, Asp71 or Glu78 of S100A9, positions Glu36, Asp67, Asn69, Asp71 and Glu78 being preferred, or
ii) Ser23, Lys26, Asn28, Ala31, Asp33, Asp59, Asn61, Asp63 and Glu70 of S100A8, position Asp33, Asp59, Asn61, Asp63 and Glu70 being preferred,
wherein the mutation preferably comprises the amino acid exchange Asn69Ala or Glu78Ala.

3. The method of any one of the preceding claims, wherein the detection method is a quantitative enzyme-linked immunosorbent assay (ELISA) comprising the following step(s):
a) pre-coating a microplate with a monoclonal capture antibody capable of binding S100A8 and/or S100A9,
b) optionally contacting the pre-coated capture antibody with the sample to be analyzed and the standard as defined in claim 1,
c) optionally washing away unbound sample and standard,
d) optionally contacting bound sample and standard with an enzyme-conjugated detecting antibody,
e) optionally washing away free amounts of the detecting antibody,
f) optionally contacting the bond detecting antibody with the substrate of the conjugated enzyme,
g) optionally finishing the enzymatic reaction,
h) optionally photometrically determining the absorbance of the sample and the standard, and
i) optionally determining the amount of S100A8/S100A9 heterodimer in the sample by comparing the absorbance with the absorbance of the standard.

4. The method of claim 3,
a) wherein the monoclonal capture antibody of step a) is capable of binding S100A8/S100A9 heterodimers but no S100A8/S100A9 tetramers, wherein the capture antibody is preferably an antibody having a binding specificity to an epitope of a vertebrate S100A9 protein, wherein the epitope has an amino acid sequence ranging from (i) amino acid position 63 to amino acid position 79 of the human protein S100A9 of Uniprot/Swissprot accession no. P06702 (SEQ ID NO: 1), or (ii) amino acid position 55 to amino acid position 71 of the human protein S100A8 of Uniprot/Swissprot accession number P05109 ( SEQ ID NO: 2); and/or
b) further comprising the step of comparing the amount of S100A8/S100A9 heterodimer determined in i) with the total amount of S100A8/S100A9 protein.

5. The method of any one of the preceding claims, wherein the sample is one of a stool sample, a blood sample, a serum sample, a plasma sample, an urine sample, a tissue extract sample or a cell culture sample, wherein said sample is preferably a sample from a subject suffering from an acute or chronic inflammatory disease, wherein said disease is preferably selected from rheumatoid arthritis, juvenile idiopathic arthritis, psoriatic arthritis, immune reconstituation inflammatory syndrome (IRIS), sepsis, systemic inflammatory response syndrome (SIRS), pneumonia, osteomyelitis, autoinflammatory syndromes, hyperzincemia, systemic inflammation, atherosclerosis, acute coronary syndrome, myocardial infarction, Crohn's disease, colitis ulcerosa, glomerulonephritis (SLE), diabetes, an inflammatory skin disease, psoriasis, inflammatory bowel disease, vasculitis, allograft rejection, glomerulonephritis, systemic lupus erythematosus, pancreatitis, a cancer, dermatomyositis and polymyositis, multiple sclerosis, allergies, autoimmune diseases, cardiovascular diseases, infections, pulmonary inflammation, systemic onset juvenile idiopathic arthritis (SOJIA), acute lung injury (ALI) and its most severe form, acute respiratory distress syndrome (ARDS).

6. A kit comprising a S100A8/S100A9 heterodimer standard as defined in claim 1, a device having an immobilized capture antibody specific for the S100A8/S100A9 heterodimer standard, a wash reagent, and a detector reagent, wherein the S100A8/S100A9 heterodimer standard comprises the amino acid exchange Asn69Ala or Glu78Ala in the amino acid sequence of S100A9.

7. Use of the S100A8/S100A9 heterodimer as a standard as defined in claim 1 in a method of detecting S100A8/S100A9 heterodimers in a sample or in a method of comparing the amount of S100A8/S100A9 heterodimers and tetramers in a sample, or use of said S100A8/S100A9 heterodimer as a standard in diagnosis or use of a kit comprising said S100A8/S100A9 heterodimer as a standard in diagnosis.

8. The use according to claim 7, wherein the use is in diagnosis and wherein the use is in a method of diagnosing an acute or chronic inflammatory disease in a human subject, wherein the diagnosis preferably comprises detection of S100A8/S100A9 heterodimer in a biological sample from said human subject.

9. A method of monitoring the progression of an acute or chronic inflammatory disease associated with an increased amount of S100A8/S100A9 heterodimer in a patient, the method comprising:
a) quantifying the amount of S100A8/S100A9 heterodimer in a sample taken from said patient by using the S100A8/S100A9 heterodimer as a standard as defined in claim 1, and
b) comparing the amount of S100A8/S100A9 heterodimer determined in a) with the amount of S100A8/S100A9 heterodimer in a sample from said patient determined at an earlier date, wherein the result of the comparison of b) provides an evaluation of the progression of the inflammatory disease associated with an increased amount of S100A8/S100A9 heterodimer in said patient.

10. The method of claim 9, wherein a significantly increased amount of S100A8/S100A9 heterodimer as compared to the reference data indicates a progression of the inflammatory disease associated with an increased amount of S100A8/S100A9 heterodimer in said patient or wherein no change or a decreased amount of S100A8/S100A9 heterodimers as compared to the reference data indicates no progression or a regression of the inflammatory disease associated with an increased amount of S100A8/S100A9 heterodimer in said patient.

11. A method of diagnosing an acute or chronic inflammatory disease in a subject, the method comprising:
a) quantifying the amount of S100A8/S100A9 heterodimer in a sample taken from said subject by using the S100A8/S100A9 heterodimer as a standard as defined in claim 1, and
b) comparing the amount of S100A8/S100A9 heterodimer as determined in a) to reference data from a subject known to not suffer from an acute or chronic inflammatory disease.

12. The method of claim 11, wherein a significant increased amount of S100A8/S100A9 heterodimer as compared to the reference data indicates that the subject suffers from an acute or chronic inflammatory disease, or wherein no significant deviation in the amount of S100A8/S100A9 heterodimer as compared to the reference data indicates that the subject does not suffer from an acute or chronic inflammatory disease.

## Patentansprüche

1. Verfahren zum Nachweis eines S100A8/S100A9-Heterodimers in einer biologischen Probe, wobei das Verfahren die Verwendung eines S100A8/S100A9-Heterodimers als Standard umfasst, das mindestens eine Mutation in mindestens einer der folgenden Regionen umfasst:
a) die hochaffine Calcium-bindende Hand von S100A9,
b) die niederaffine Calcium-bindende Hand von S100A9,
c) die hochaffine Calcium-bindende Hand von S100A8, oder
d) die niederaffine Calcium-bindende Hand von S100A8,
wobei der S100A8/S100A9-Heterodimer-Standard nicht zu (S100A8/S100A9)₂-Tetrameren tetramerisiert.

2. Verfahren nach Anspruch 1, wobei der S100A8/S100A9-Heterodimer-Standard mindestens eine Mutation aufweist in:
a) der Aminosäuresequenz im Bereich von Aminosäureposition 63 bis Aminosäureposition 79 des menschlichen S100A9-Proteins der Uniprot/Swissprot-Hinterlegungsnummer P06702 (SEQ ID NO: 1),
b) der Aminosäuresequenz im Bereich von Aminosäureposition 20 bis Aminosäureposition 38 des menschlichen S100A9-Proteins der Uniprot/Swissprot-Hinterlegungsnummer P06702 (SEQ ID NO: 1),
c) der Aminosäuresequenz im Bereich von Aminosäureposition 55 bis Aminosäureposition 71 des menschlichen S100A8-Proteins der Uniprot/Swissprot-Hinterlegungsnummer P05109 (SEQ ID NO: 2) oder
d) der Aminosäuresequenz im Bereich von Aminosäureposition 20 bis Aminosäureposition 38 des menschlichen S100A8-Proteins der Uniprot/Swissprot-Hinterlegungsnummer P05109 (SEQ ID NO: 2), und Kombinationen davon,
wobei der S100A8/S100A9-Heterodimer-Standard vorzugsweise mindestens eine Mutation umfasst, ausgewählt aus:
i) Ser23, Leu26, His28, Thr31, Asp 33, Glu36, Asp67, Asn69, Asp71 oder Glu78 von S100A9, wobei die Positionen Glu36, Asp67, Asn69, Asp71 und Glu78 bevorzugt sind, oder
ii) Ser23, Lys26, Asn28, Ala31, Asp33, Asp59, Asn61, Asp63 und Glu70 von S100A8, wobei die Positionen Asp33, Asp59, Asn61, Asp63 und Glu70 bevorzugt sind,
wobei die Mutation vorzugsweise den Aminosäureaustausch Asn69Ala oder Glu78Ala umfasst.

3. Verfahren nach einem der vorhergehenden Ansprüche, wobei das Nachweisverfahren ein quantitativer Enzym-linked Immunosorbent Assay (ELISA) ist, der den/die folgenden Schritt(e) umfasst:
a) Vorbeschichten einer Mikroplatte mit einem monoklonalen Fänger-Antikörper, der S100A8 und/oder S100A9 binden kann,
b) gegebenenfalls Kontaktieren des vorbeschichteten Fänger-Antikörpers mit der zu analysierenden Probe und dem Standard wie in Anspruch 1 definiert,
c) gegebenenfalls Abwaschen von ungebundener Probe und Standard,
d) gegebenenfalls Kontaktieren der gebundener Probe und Standard mit einem Enzym-konjugierten Nachweis-Antikörper,
e) gegebenenfalls Abwaschen freier Mengen des Nachweis-Antikörpers,
f) gegebenenfalls Kontaktieren des gebundenen Nachweis-Antikörpers mit dem Substrat des konjugierten Enzyms,
g) gegebenenfalls Beendigung der enzymatischen Reaktion,
h) gegebenenfalls photometrische Bestimmung der Extinktion der Probe und des Standards und
i) gegebenenfalls Bestimmung der Menge des S100A8/S100A9-Heterodimers in der Probe durch Vergleich der Extinktion mit der Extinktion des Standards.

4. Verfahren nach Anspruch 3,
a) wobei der monoklonale Fänger-Antikörper von Schritt a) in der Lage ist, S100A8/S100A9-Heterodimere, aber keine S100A8/S100A9-Tetramere zu binden, wobei der Fänger-Antikörper vorzugsweise ein Antikörper mit einer Bindungsspezifität für ein Epitop eines Wirbeltier-S100A9-Proteins ist, wobei das Epitop eine Aminosäuresequenz aufweist, die von (i) Aminosäureposition 63 bis Aminosäureposition 79 des menschlichen S100A9-Proteins der Uniprot/Swissprot-Hinterlegungsnummer P06702 (SEQ ID NO: 1) oder (ii) Aminosäureposition 55 bis Aminosäureposition 71 des menschlichen S100A8-Proteins der Uniprot/Swissprot-Hinterlegungsnummer P05109 (SEQ ID NO: 2) reicht; und/oder
b) ferner umfassend den Schritt des Vergleichens der in i) bestimmten Menge an S100A8/S100A9-Heterodimer mit der Gesamtmenge an S100A8/S100A9-Protein.

5. Verfahren nach einem der vorhergehenden Ansprüche, wobei es sich bei der Probe um eine Stuhlprobe, eine Blutprobe, eine Serumprobe, eine Plasmaprobe, eine Urinprobe, eine Gewebeextraktprobe oder eine Zellkulturprobe handelt, wobei die Probe vorzugsweise von einem Subjekt stammt, das an einer akuten oder chronischen entzündlichen Erkrankung leidet, wobei die Krankheit vorzugsweise ausgewählt ist aus rheumatoider Arthritis, juveniler idiopathischer Arthritis, psoriatischer Arthritis, entzündlichem Immunrekonstruktionssyndrom (IRIS), Sepsis, systemischem Entzündungsreaktionssyndrom (SIRS), Lungenentzündung, Osteomyelitis, autoinflammatorischen Syndromen, Hyperzinkämie, systemischer Entzündung, Arteriosklerose, akutem Koronarsyndrom, Myokardinfarkt, Morbus Crohn, Colitis ulcerosa, Glomerulonephritis (SLE), Diabetes, einer entzündlichen Hauterkrankung, Psoriasis, entzündlicher Darmerkrankung, Vaskulitis, Abstoßung von Allotransplantaten, Glomerulonephritis, systemischer Lupus erythematodes, Pankreatitis, Krebs, Dermatomyositis und Polymyositis, Multiple Sklerose, Allergien, Autoimmunerkrankungen, Herz-Kreislauf-Erkrankungen, Infektionen, Lungenentzündungen, systemisch auftretender juveniler idiopathischer Arthritis (SOJIA), akuter Lungenschädigung (ALI) und ihrer schwersten Form, akutem Atemnotsyndrom (ARDS).

6. Kit, das einen S100A8/S100A9-Heterodimer-Standard wie in Anspruch 1 definiert, eine Vorrichtung mit einem immobilisierten Fänger-Antikörper, der spezifisch für den S100A8/S100A9-Heterodimer-Standard ist, ein Waschreagenz und ein Detektorreagenz umfasst, wobei der S100A8/S100A9-Heterodimer-Standard den Aminosäureaustausch Asn69Ala oder Glu78Ala in der Aminosäuresequenz von S100A9 umfasst.

7. Verwendung des S100A8/S100A9-Heterodimers als Standard wie in Anspruch 1 definiert in einem Verfahren zum Nachweis von S100A8/S100A9-Heterodimeren in einer Probe oder in einem Verfahren zum Vergleich der Menge von S100A8/S100A9-Heterodimeren und -Tetrameren in einer Probe, oder Verwendung des S100A8/S100A9-Heterodimers als Standard in der Diagnose oder Verwendung eines Kits, das dieses S100A8/S100A9-Heterodimer als Standard umfasst, in der Diagnose.

8. Verwendung nach Anspruch 7, wobei die Verwendung in der Diagnose erfolgt und wobei die Verwendung in einem Verfahren zur Diagnose einer akuten oder chronischen entzündlichen Erkrankung in einem menschlichen Subjekt erfolgt, wobei die Diagnose vorzugsweise den Nachweis von S100A8/S100A9-Heterodimer in einer biologischen Probe von dem menschlichen Subjekt umfasst.

9. Verfahren zur Überwachung des Fortschreitens einer akuten oder chronischen entzündlichen Erkrankung, die mit einer erhöhten Menge an S100A8/S100A9-Heterodimer in einem Patienten verbunden ist, wobei das Verfahren umfasst:
a) Quantifizieren der Menge an S100A8/S100A9-Heterodimer in einer Probe, die dem Patienten entnommen wurde, unter Verwendung des S100A8/S100A9-Heterodimers als Standard wie in Anspruch 1 definiert, und
b) Vergleichen der Menge an S100A8/S100A9-Heterodimer, die in a) bestimmt wurde, mit der Menge an S100A8/S100A9-Heterodimer in einer Probe von dem Patienten, die zu einem früheren Zeitpunkt bestimmt wurde, wobei das Ergebnis des Vergleichs von b) eine Bewertung des Fortschreitens der entzündlichen Erkrankung, die mit einer erhöhten Menge an S100A8/S100A9-Heterodimer in dem Patienten verbunden ist, liefert.

10. Verfahren nach Anspruch 9, wobei eine signifikant erhöhte Menge an S100A8/S100A9-Heterodimer im Vergleich zu den Referenzdaten auf ein Fortschreiten der entzündlichen Erkrankung, die mit einer erhöhten Menge an S100A8/S100A9-Heterodimer bei dem Patienten assoziiert ist, hinweist oder wobei keine Veränderung oder eine verringerte Menge an S100A8/S100A9-Heterodimer im Vergleich zu den Referenzdaten auf kein Fortschreiten oder eine Regression der entzündlichen Erkrankung, die mit einer erhöhten Menge an S100A8/S100A9-Heterodimer bei dem Patienten assoziiert ist, hinweist.

11. Verfahren zur Diagnose einer akuten oder chronischen entzündlichen Erkrankung bei einem Subjekt, wobei das Verfahren umfasst:
a) Quantifizieren der Menge an S100A8/S100A9-Heterodimer in einer Probe, die dem Subjekt entnommen wurde, unter Verwendung des S100A8/S100A9-Heterodimers als Standard wie in Anspruch 1 definiert, und
b) Vergleichen der Menge an S100A8/S100A9-Heterodimer, wie in a) bestimmt, mit Referenzdaten von einem Subjekt, von dem bekannt ist, dass es nicht an einer akuten oder chronischen entzündlichen Krankheit leidet.

12. Verfahren nach Anspruch 11, wobei eine signifikant erhöhte Menge an S100A8/S100A9-Heterodimer im Vergleich zu den Referenzdaten anzeigt, dass das Subjekt an einer akuten oder chronischen entzündlichen Erkrankung leidet, oder wobei keine signifikante Abweichung in der Menge an S100A8/S100A9-Heterodimer im Vergleich zu den Referenzdaten anzeigt, dass das Subjekt nicht an einer akuten oder chronischen entzündlichen Erkrankung leidet.

## Revendications

1. Méthode de détection d'un hétérodimère S100A8/S100A9 dans un échantillon biologique, le procédé comprenant l'utilisation d'un hétérodimère S100A8/S100A9 comme étalon qui comporte au moins une mutation dans au moins une des régions suivantes:
a) la main de liaison au calcium à haute affinité de S100A9,
b) la main de liaison au calcium à faible affinité de S100A9,
c) la main de liaison au calcium de haute affinité de S100A8, ou
d) la main de liaison au calcium à faible affinité de S100A8,
dans lequel l'hétérodimère standard S100A8/S100A9 ne se tétramérise pas en tétramères (S100A8/S100A9)₂.

2. La méthode de la revendication 1, dans laquelle l'hétérodimère standard S100A8/S100A9 comprend au moins une mutation dans:
a) la séquence d'acides aminés allant de la position 63 à la position 79 de la protéine humaine S100A9 du numéro d'accès Uniprot/Swissprot P06702 (SEQ ID NO: 1),
b) la séquence d'acides aminés allant de la position 20 à la position 38 de la protéine humaine S100A9 du numéro d'accès Uniprot/Swissprot P06702 (SEQ ID NO: 1),
c) la séquence d'acides aminés allant de la position 55 à la position 71 de la protéine humaine S100A8 du numéro d'accès Uniprot/Swissprot P05109 (SEQ ID NO: 2), ou
d) la séquence d'acides aminés allant de la position 20 à la position 38 de la protéine humaine S100A8 du numéro d'accès Uniprot/Swissprot P05109 (SEQ ID NO: 2), et leurs combinaisons,
dans lequel l'hétérodimère standard S100A8/S100A9 comprend de préférence au moins une mutation choisie parmi:
i) Ser23, Leu26, His28, Thr31, Asp 33, Glu36, Asp67, Asn69, Asp71 ou Glu78 de S100A9, les positions Glu36, Asp67, Asn69, Asp71 et Glu78 étant préférées, ou
ii) Ser23, Lys26, Asn28, Ala31, Asp33, Asp59, Asn61, Asp63 et Glu70 de S100A8, les positions Asp33, Asp59, Asn61, Asp63 et Glu70 étant préférées,
dans lequel la mutation comprend de préférence l'échange d'acides aminés Asn69Ala ou Glu78Ala.

3. La méthode de l'une quelconque des revendications précédentes, dans lequel la méthode de détection est un dosage immuno-enzymatique quantitatif (ELISA) comprenant la ou les étapes suivantes:
a) pré-endossement d'une microplaque avec un anticorps monoclonal de capture capable de lier la S100A8 et/ou la S100A9,
b) mettre éventuellement en contact l'anticorps de capture précouché avec l'échantillon à analyser et l'étalon tel que défini dans la revendication 1,
c) lavage éventuel de l'échantillon et de l'étalon non liés,
d) éventuellement, mise en contact de l'échantillon et de l'étalon liés avec un anticorps de détection conjugué à une enzyme,
e) lavage éventuel des quantités libres de l'anticorps de détection,
f) éventuellement, mise en contact de l'anticorps de détection de liaison avec le substrat de l'enzyme conjuguée,
g) éventuellement, terminer la réaction enzymatique,
h) déterminer éventuellement par photométrie l'absorbance de l'échantillon et de l'étalon, et
i) déterminer éventuellement la quantité d'hétérodimère S100A8/S100A9 dans l'échantillon en comparant l'absorbance avec l'absorbance de l'étalon.

4. La méthode de la revendication 3,
a) dans laquelle l'anticorps monoclonal de capture de l'étape a) est capable de lier les hétérodimères S100A8/S100A9 mais pas les tétramères S100A8/S100A9, dans laquelle l'anticorps de capture est de préférence un anticorps ayant une spécificité de liaison à un épitope d'une protéine S100A9 de vertébré, dans laquelle l'épitope a une séquence d'acides aminés allant de (i) la position d'acide aminé 63 à la position d'acide aminé 79 de la protéine humaine S100A9 du numéro d'accès Uniprot/Swissprot P06702 (SEQ ID NO : 1), ou (ii) de la position d'acide aminé 55 à la position d'acide aminé 71 de la protéine humaine S100A8 du numéro d'accès Uniprot/Swissprot P05109 (SEQ ID NO: 2); et/ou
b) comprenant en outre l'étape consistant à comparer la quantité d'hétérodimère S100A8/S100A9 déterminée en i) à la quantité totale de protéine S100A8/S100A9.

5. La méthode de l'une des revendications précédentes, dans laquelle l'échantillon est un échantillon de selles, un échantillon de sang, un échantillon de sérum, un échantillon de plasma, un échantillon d'urine, un échantillon d'extrait de tissu ou un échantillon de culture cellulaire, dans lequel ledit échantillon est de préférence un échantillon provenant d'un sujet souffrant d'une maladie inflammatoire aiguë ou chronique, dans laquelle ladite maladie est de préférence choisie parmi l'arthrite rhumatoïde, l'arthrite juvénile idiopathique, l'arthrite psoriasique, le syndrome inflammatoire de reconstitution immunitaire (IRIS), la septicémie, le syndrome de réponse inflammatoire systémique (SIRS), la pneumonie, l'ostéomyélite, les syndromes auto-inflammatoires, l'hyperzincémie, inflammation systémique, athérosclérose, syndrome coronarien aigu, infarctus du myocarde, maladie de Crohn, colite ulcéreuse, glomérulonéphrite (LED), diabète, maladie inflammatoire de la peau, psoriasis, maladie inflammatoire de l'intestin, vascularite, rejet d'allogreffe, glomérulonéphrite, lupus érythémateux disséminé, pancréatite, cancer, dermatomyosite et polymyosite, sclérose en plaques, allergies, maladies auto-immunes, maladies cardiovasculaires, infections, inflammations pulmonaires, arthrite juvénile idiopathique d'apparition systémique (SOJIA), lésions pulmonaires aiguës (LPA) et sa forme la plus grave, le syndrome de détresse respiratoire aiguë (SDRA).

6. Kit comprenant un standard d'hétérodimère S100A8/S100A9 tel que défini dans la revendication 1, un dispositif ayant un anticorps de capture immobilisé spécifique pour le standard d'hétérodimère S100A8/S100A9, un réactif de lavage, et un réactif de détection, dans lequel le standard d'hétérodimère S100A8/S100A9 comprend l'échange d'acide aminé Asn69Ala ou Glu78Ala dans la séquence d'acide aminé de S100A9.

7. Utilisation de l'hétérodimère S100A8/S100A9 comme étalon tel que défini dans la revendication 1 dans une méthode de détection des hétérodimères S100A8/S100A9 dans un échantillon ou dans une méthode de comparaison de la quantité d'hétérodimères et de tétramères S100A8/S100A9 dans un échantillon, ou utilisation dudit hétérodimère S100A8/S100A9 comme étalon dans le diagnostic ou utilisation d'un kit comprenant ledit hétérodimère S100A8/S100A9 comme étalon dans le diagnostic.

8. Utilisation selon la revendication 7, dans laquelle l'utilisation est diagnostique et dans laquelle l'utilisation est une méthode de diagnostic d'une maladie inflammatoire aiguë ou chronique chez un sujet humain, dans laquelle le diagnostic comprend de préférence la détection de l'hétérodimère S100A8/S100A9 dans un échantillon biologique dudit sujet humain.

9. Méthode de suivi de l'évolution d'une maladie inflammatoire aiguë ou chronique associée à une augmentation de l'hétérodimère S100A8/S100A9 chez un patient, la méthode comprenant:
a) quantifier la quantité d'hétérodimère S100A8/S100A9 dans un échantillon prélevé sur ledit patient en utilisant l'hétérodimère S100A8/S100A9 comme étalon tel que défini dans la revendication 1, et
b) comparer la quantité d'hétérodimère S100A8/S100A9 déterminée en a) avec la quantité d'hétérodimère S100A8/S100A9 dans un échantillon dudit patient déterminé à une date antérieure, le résultat de la comparaison en b) fournissant une évaluation de la progression de la maladie inflammatoire associée à une quantité accrue d'hétérodimère S100A8/S100A9 chez ledit patient.

10. La méthode de la revendication 9, dans laquelle une quantité significativement accrue d'hétérodimères S100A8/S100A9 par rapport aux données de référence indique une progression de la maladie inflammatoire associée à une quantité accrue d'hétérodimères S100A8/S100A9 chez ledit patient ou dans laquelle aucun changement ou une quantité réduite d'hétérodimères S100A8/S100A9 par rapport aux données de référence indique l'absence de progression ou une régression de la maladie inflammatoire associée à une quantité accrue d'hétérodimères S100A8/S100A9 chez ledit patient.

11. Méthode de diagnostic d'une maladie inflammatoire aiguë ou chronique chez un sujet, la méthode comprenant:
a) quantifier la quantité d'hétérodimères S100A8/S100A9 dans un échantillon prélevé sur ledit sujet en utilisant l'hétérodimère S100A8/S100A9 comme étalon tel que défini dans la revendication 1, et
b) comparer la quantité d'hétérodimère S100A8/S100A9 déterminée en a) à des données de référence provenant d'un sujet connu pour ne pas souffrir d'une maladie inflammatoire aiguë ou chronique.

12. La méthode de la revendication 11, dans laquelle une augmentation significative de la quantité d'hétérodimères S100A8/S100A9 par rapport aux données de référence indique que le sujet souffre d'une maladie inflammatoire aiguë ou chronique, ou dans laquelle aucune déviation significative de la quantité d'hétérodimères S100A8/S100A9 par rapport aux données de référence indique que le sujet ne souffre pas d'une maladie inflammatoire aiguë ou chronique.
